(19) Europäisches Patentamt

European Patent Office

Office européen des brevets

(11) EP 0 624 565 B1

(12) **EUROPÄISCHE PATENTSCHRIFT**

(45) Veröffentlichungstag und Bekanntmachung des Hinweises auf die Patenterteilung:
**15.01.1997 Patentblatt 1997/03**

(51) Int. Cl.$^6$: **C07C 45/38**, C07C 45/29, C07C 47/04

(21) Anmeldenummer: **94106963.5**

(22) Anmeldetag: **04.05.1994**

(54) **Verfahren zur Herstellung von Formaldehyd durch oxidative Dehydrierung von Methanol in Gegenwart von Distickstoffoxid**

Process for the preparation of formaldehyde by oxidative dehydrogenation of methanol in the presence of nitrous oxide

Procédé de préparation de formaldéhyde par déshydrogénation oxydante de méthanol en présence d'oxyde nitreux

(84) Benannte Vertragsstaaten:
**BE DE DK ES FR GB IT NL PT SE**

(30) Priorität: **12.05.1993 DE 4315799**

(43) Veröffentlichungstag der Anmeldung:
**17.11.1994 Patentblatt 1994/46**

(73) Patentinhaber: **BASF Aktiengesellschaft 67063 Ludwigshafen (DE)**

(72) Erfinder:
- **Diercks, Rainer, Dr.**
  **D-67141 Neuhofen (DE)**
- **Essig, Manfred, Dr.**
  **D-67697 Otterberg (DE)**
- **Marosi, Laszlo, Dr.**
  **D-67063 Ludwigshafen (DE)**

(56) Entgegenhaltungen:
**FR-A- 1 487 093          US-A- 4 233 248**
**US-A- 4 517 392**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung von Formaldehyd durch oxidative Dehydrierung von Methanol mittels eines sauerstoffhaltigen Gases in der Gasphase an einem Silber- oder silberhaltigen Katalysator.

Es ist allgemein bekannt, Formaldehyd durch oxidative Dehydrierung von Methanol in Gegenwart von Silberkatalysatoren bei erhöhten Temperaturen herzustellen (Ullmanns Encyklopädie der Technischen Chemie, 4. Auflage, Band 11, S. 611 ff.), wobei am Silberkontakt im wesentlichen folgende Reaktionen ablaufen:

$$CH_3OH + 1/2\ O_2 \rightarrow CH_2O + H_2O$$

$$CH_3OH \Leftrightarrow CH_2O + H_2$$

$$H_2 + 1/2\ O_2 \rightarrow H_2O$$

Bei bekannten Verfahren besteht hinsichtlich der Ausbeute noch Spielraum für Verbesserungen. Es hat verschiedene Ansätze zur Ausbeutesteigerung gegeben.

So wird in der GB-A 21 21 787 der Einsatz spezieller Silber-/Palladiumlegierungen beschrieben, wobei jedoch bei dieser Verfahrensweise eine einfache elektrolytische Katalysatorregenerierung Schwierigkeiten bereitet.

Nach der DE-A 24 42 231 wird Methanol im Gemisch mit Wasserdampf und Luft an einem Silberkatalysator zu Formaldehyd umgesetzt, wobei ein Teil des Reaktionsabgases wieder zurückgeführt werden soll.

Aus der US-A 4 223 248 ist ein Verfahren zur Herstellung von Formaldehyd aus Methanol an einem silberhaltigen Katalysator bekannt, bei dem als oxidierendes Gas ausschließlich Distickstoffoxid eingesetzt wird.

Nachteilig an diesen Verfahren ist jedoch, daß sie hinsichtlich der Ausbeuten noch nicht als befriedigend angesehen werden können oder hinsichtlich der Abgasaualitäten und Restmethanolgehalte noch Verbesserungen zulassen oder nur unter verfahrenstechnischem Aufwand realisiert werden können.

Die Aufgabe der vorliegenden Erfindung war nun, ein verbessertes Verfahren zu finden, welches auf einfache Weise eine Steigerung von Selektivität und Ausbeute ermöglicht und die Katalysatoraufarbeitung nicht beeinflußt.

Demgemäß wurde ein Verfahren zur Herstellung von Formaldehyd durch oxidative Dehydrierung von Methanol mittels eines sauerstoffhaltigen Gases in der Gasphase an einem Silber- oder silberhaltigen Katalysator gefunden, welches dadurch gekennzeichnet ist, daß das sauerstoffhaltige Gas 1 bis 50 Vol-% Distickstoffoxid enthält.

Für das Verfahren geeignete Ausgangsstoffe sind reines Methanol, technisches Methanol, Rohmethanol oder vorteilhaft deren Mischungen mit Wasser; die Methanolkonzentration der wäßrigen Gemische beträgt zweckmäßigerweise 40 bis 95 Gew.-%, vorzugsweise 50 bis 85 Gew.-%. Das Methanol wird dem Reaktorraum in Dampfform zugeführt, vorteilhaft im Gemisch mit Wasserdampf und gegebenenfalls einem Inertgas. Als Inertgas kommt beispielsweise Stickstoff in Betracht.

Als oxidierendes Agens wird ein sauerstoffhaltiges Gas verwendet. Erfindungsgemäß verwendet man ein Gemisch aus 99 bis 50 Vol-% Luft und 1 bis 50 Vol-% Distickstoffoxid ($N_2O$). Bevorzugt werden Volumenanteile an $N_2O$ von 2 bis 30 %, besonders bevorzugt 7 bis 25 %. Die Mengen an $N_2O$ sind dabei so zu wählen, daß ein Molverhältnis von Methanol zu $N_2O$ im Bereich von 55:1 bis 3,5:1, bevorzugt 15:1 bis 4,0:1, besonders bevorzugt 7:1 bis 4,5:1 vorliegt.

$N_2O$ steht als unerwünschtes Nebenprodukt aus einer Reihe von großtechnischen Verfahren zur Verfügung, wie z.B. der Adipinsäure- oder der Hydroxylaminsynthese, wo es entsorgt werden muß. Die vorliegende Erfindung erlaubt nun den gezielten Einsatz von $N_2O$ bei einem technischen Großverfahren.

Aus verfahrenstechnischen Gründen kann es sich empfehlen, das $N_2O$ im Gemisch mit Stickstoff der Ausgangsstoffmischung zuzuführen.

Die Ausgangsstoffe werden in an sich üblicher Weise durch einen Silber enthaltenden Festbettkatalysator geleitet, der in einen senkrecht stehenden Rohrreaktor eingebracht ist. Der Katalysator besteht bevorzugt aus Silberkristallen mit einer Korngröße von 0,1 bis 3 mm, insbesondere von 0,2 bis 2,5 mm. Der Festbettkatalysator kann durch Anordnung der Silberkristalle in Schichten mit unterschiedlichen Korngrößen einen Mehrschichtaufbau aufweisen. Bevorzugt weist die Katalysatorschicht einen Mehrschichtaufbau auf, wie er in der DE-B 23 22 757 beschrieben ist.

Das Ausgangsgemisch aus Methanoldampf, sauerstoffhaltigem Gas, $N_2O$ und gegebenenfalls Wasserdampf und Inertgas wird im Rohrreaktor bevorzugt von oben nach unten geführt.

Das Verfahren wird im übrigen in an sich bekannter Weise einstufig durchgeführt, indem man das Ausgangsgemisch bei Temperaturen im Bereich von 550 bis 750°C, insbesondere 600 bis 720°C, besonders vorteilhaft 660 bis 700°C, durch das Katalysatorfestbett leitet. Das Verfahren wird im allgemeinen bei einem Druck zwischen 0,5 und 3 bar, insbesondere 0,8 bis 2 bar, bevorzugt 1 bis 1,5 bar, kontinuierlich durchgeführt. Die Verweilzeiten in der Katalysatorzone liegen im Bereich von 0,001 bis 1 Sekunde, bevorzugt zwischen 0,002 und 0,1 Sekunden. Vorteilhafterweise werden die die Katalysatorzone verlassenden Reaktionsgase innerhalb kurzer Zeit abgekühlt, z.B. auf Temperaturen kleiner 350°C. Das abgekühlte Gasgemisch kann dann zweckmäßigerweise einem Adsorptionsturm zugeführt werden, in welchem der Formaldehyd mit Wasser aus dem Gasgemisch ausgewaschen wird.

Das erfindungsgemäße Verfahren zur Herstellung von Formaldehyd führt auf einfache Weise zu vorteilhaften Ausbeuten, bei hohen Umsätzen und hoher Selektivität. Weiterhin wird durch das erfindungsgemäße Verfahren die Oxidation des bei der thermischen Dehydrierung entstehenden Wasserstoffs zu Wasser zurückgedrängt, was zu höheren Wasserstoffgehalten im Abgas und damit zu besseren Energieausbeuten bei der Verbrennung führt oder höheren Wasserstoffmengen bei der $H_2$-Abtrennung.

Vorteilhaft ist auch, daß die Restmethanolgehalte gesenkt werden können, wodurch sich für eine Reihe von Anwendungen eine destillative Methanolabreicherung erübrigt. Zudem kann als unerwünschtes Nebenprodukt vorhandenes $N_2O$ genutzt werden, was einen weiteren wirtschaftlichen Vorteil bietet.

In einer bestehenden Anlage wurden die folgenden Versuche durchgeführt, wobei zur Konstanthaltung der Abgasmenge, auf welche die Anlage ausgelegt wurde, Stickstoff zugeführt wurde.

Beispiele 1 bis 4

Es wurde ein senkrecht stehender Reaktor mit einem Innendurchmesser von 2 cm verwendet, in dessen oberem Teil ein dreischichtiges Katalysatorfestbett von insgesamt 10 mm Höhe angebracht war.

Die untere Schicht bestand aus 4 g Silber der Korngröße 0,75 mm bis 1,0 mm, die mittlere Schicht aus 7 g Silber der Korngröße von 0,4 mm bis 0,75 mm und die obere Schicht aus 3 g Silber der Korngröße 0,2 bis 0,4 mm.

Pro Stunde wurde ein Gemisch aus 300 g Methanol und 200 g Wasser in Dampfform sowie den in der Tabelle angegebenen Mengen Luft bzw. Luft/$N_2O$ von oben nach unten durch das Katalysatorbett geleitet. Die Umsetzung erfolgte bei einer Temperatur von 660°C in der Katalysatorzone und einem Druck von 1,1 bar. Im Anschluß an die Umsetzung wurde das Reaktionsgemisch auf 150°C abgekühlt und in Wasser aufgenommen.

| Beispiel Nr. | 1 | 2 | 3 | 4 |
|---|---|---|---|---|
| Luft [l/h] | 380 | 335 | 290 | 245 |
| $N_2O$ [l/h] | - | 15 | 30 | 45 |
| Umsatz [%] | 97,9 | 98,5 | 98,9 | 99,1 |
| Selektivität [%] | 92,2 | 92,9 | 93,4 | 94,1 |
| Ausbeute [%] | 90,2 | 91,5 | 92,4 | 93,3 |
| $H_2$-Gehalt im Abgas [1]) [Vol-%] | 18,0 | 20,2 | 21,8 | 24,3 |
| Rest-Methanolgehalt [2]) [%] | 2,3 | 1,5 | 1,3 | 1,1 |

[1]) Abgasvolumen durch $N_2$-Zugabe auf 390 l konstant gehalten
[2]) bezogen auf Formaldehyd

**Patentansprüche**

1. Verfahren zur Herstellung von Formaldehyd durch oxidative Dehydrierung von Methanol in der Gasphase an einem Silber- oder silberhaltigen Katalysator mittels eines sauerstoffhaltigen Gases, dadurch gekennzeichnet, daß das sauerstoffhaltige Gas 1 bis 50 Vol-% Distickstoffoxid enthält.

**Claims**

1. A process for the preparation of formaldehyde by oxidative dehydrogenation of methanol in the gas phase on a silver or silver-containing catalyst by means of an oxygen-containing gas which contains from 1 to 50% by volume of dinitrogen oxide.

**Revendications**

1. Procédé de préparation du formaldéhyde par la déshydrogénation oxydante du méthanol, en phase gazeuse, sur un catalyseur à argent ou contenant de l'argent, à l'aide d'un gaz contenant de l'oxygène, caractérisé en ce que le gaz contenant de l'oxygène comporte 1 à 50% en volume de monoxyde de diazote ou hémioxyde d'azote ou oxyde nitreux.